# EUROPEAN PATENT SPECIFICATION

(11) **EP 1 460 995 B1**
(45) Date of publication and mention of the grant of the patent: **10.11.2010**
(21) Application number: 02784914.0
(22) Date of filing: 13.12.2002
(51) Int. Cl.: A61K 6/00, A61K 9/00, A61K 47/18

(54) **TRANSDERMAL TRANSPORT OF COMPOUNDS**
TRANSDERMALER TRANSPORT VON VERBINDUNGEN
TRANSPORT TRANSDERMIQUE DE COMPOSES

(30) Priority: 13.12.2001 AU PR946301; 09.08.2002 AU 2002950711
(43) Date of publication of application: 29.09.2004
(73) Proprietor: Vital Health Sciences Pty Ltd., Melbourne, VIC 3000 (AU)
(72) Inventor: WEST, Simon, Michael, Williamstown, VIC 3016 (AU); KANNAR, David, Belgrave South, VIC 3160 (AU)
(74) Representative: Vossius & Partner
(86) International application number: PCT/AU2002/001686
(87) International publication number: WO 2003/049774

(56) References cited:
- EP-B1- 0 574 255
- WO-A-00/59475
- WO-A-00/71125
- WO-A-91/17987
- WO-A-92/08459
- WO-A-93/24131
- WO-A-95/34303
- WO-A-96/20715
- WO-A-96/21440
- WO-A-96/29336
- WO-A-97/35591
- WO-A1-02/40034
- US-A- 5 374 645
- US-A- 5 446 070
- DATABASE WPI Week 199413, Derwent Publications Ltd., London, GB; AN 1994-103911, XP003019035 & JP 6 048 962 A (MORIMOTO Y) 22 February 1994
- DATABASE BIOSIS [Online] BIOSCIENCES INFORMATION SERVICE, PHILADELPHIA, PA, US; PREV198579102664 THURESSON-KLEIN A. ET AL: "Estrogen stimulates formation of lamellar bodies and release of surfactant in the rat fetal lung"
- SCHUBERT K. ET AL: "Vergleichende Stoffwechsel-Untersuchungen nach Infusion von Delta-4-Androsten-3,17-dion, Testosteron-Phosphat und Dehydroepiandrostenon-Phosphat" ENDOKRINOLOGIE, vol. 49, no. 1, 1965, pages 1-12, ISSN: 0013-7251

## Description

### Field of the Invention

This invention relates to transdermal drug transport for the application of drug compounds and methods for improving dermal penetration of compounds.

### Background of the Invention

Therapeutic substances have been applied topically to the skin for many years. First reports date back to the Egyptians who applied ointments to the skin for healing. However, it was only in the late nineteenth century that some compounds were discovered to be better absorbed through the skin than others. Since then, scientists have worked to determine the mechanisms controlling percutaneous absorption and how to manipulate these routes.

The skin is the largest organ of the body, which functions to protect the internal organs from external chemical, physical and pathological hazards. Normal skin is divided into three layers: the epidermis, the dermis, and subcutaneous tissue. The outer cornified layer of the epidermis, the stratum corneum, possesses properties of strength, flexibility, high electrical impedance and dryness that retards penetration and proliferation of microorganisms. The stratum corneum is also the principle barrier to transdermal drug absorption. There is a layer of sebum protecting the skin which has not commonly been considered to be a barrier to drug transport.

When travelling through the skin, a diffusing drug molecule has a number of potential transport routes available. These include appendageal such as eccrine, follicular or epidermal such as inter or intra cellular. Current theories regarding the transport route point to two possible mechanisms: (i) passive transcellular and (ii) intracellular epidermal transport. Evidence also exists that active transport systems in the skin are important to regulate homeostasis and that this process operates to move compounds across membranes and throughout the dermis. Active transport mechanisms have not been considered or exploited as a drug delivery pathway, as the absorption process is believed to be largely a permeation process.

Drugs are topically applied in a number of ways including ointments, patches, solutions, subcutaneous depots, poultices, plasters and transdermal delivery devices. Transdermal delivery devices are a subset of topical application which involves the drug penetrating the dermis in contrast to a mere topical application in which a drug acts on the surface of the dermis.

Transdermal delivery devices include diffusion controlled polymeric membrane reservoir delivery systems. Such devices, often in the form of patches, aim to maintain a constant rate of drug release over an extended period of time under steady state conditions, which is sometimes referred to as "zero-order release" or "zero-order kinetics". Such patches usually consist of one or more rate controlling membranes surrounding a drug solution operating to maintain a constant rate of drug release. This is generally achieved by using membrane devices, microcapsules, liposomes and hollow fibres in various polymeric materials including silicone rubber, ethylene vinyl acetate, cellulose acetate, copolymers, polyurethanes and hydrogels.

Transdermal delivery has been recognized to offer several potential benefits including achieving similar blood levels as slow intravenous infusion without the inconvenience, better control of absorption and metabolism compared to oral administration, continuity of drug effect especially of drugs with short half lives, equivalent efficacy with reduced drug dosage by by-passing hepatic first pass elimination, lower risk of under or overdosing and better patient compliance through simplified dosage regime.

Transdermal delivery devices are useful for drugs that are difficult to deliver orally, have short half lives or need zero order. They are currently utilized to deliver drugs in a number of therapeutic areas ranging from but not limited to sex hormones in birth control and management of the menopause (Estraderm® by Ciba-Geigy, Oestradiol implants by Organon), gonadal hormones in anticancer therapy (Depo-Provera® by Pharmacia-Upjohn) analgesic subcutaneous depots in chronic pain control, nitrates in angina therapy (Deponit® by Pharma- Schwarz/Lohmann, Nitrodisc® by Searle, Nitro-Dur® by Key, .Transiderm- Nitro® by Ciba-Geigy, Frandol® by Yamanouchi), antiemetics in motion sickness and nausea (Scop® by Ciba-Geigy), and nicotine in smoking cessation therapy (Nicabate® by Marion Merrell Dow, Nicotinell® by Ciba-Geigy).

Transdermal estradiol administration is a well established method for treating menopausal symptoms and hypogonadal conditions in adolescent females, but skin irritation has been reported in up to 17% of females using the patches. Transdermal administration is preferred to oral administration because it avoids hepatic first-pass metabolism as well as issues of metabolization and absorbance through intestinal walls. In addition, lower dosages can be administered compared to those used in oral therapy. Ideally, a physiologically active metabolite of estrogen would improve bioactivity, however, their highly lipidic nature makes itextremely difficult to deliver.

A study by Morgan, TM. , Parr, RA. , Reed, BL. and Finnin, BC (1998). "Enhanced transdermal delivery of sex hormones in swine with a novel topical aerosol" J. Pharm. Sci. 87 (10): 1219-1225 investigated the transdermal delivery of testosterone and estradiol in pigs using a novel metered dose topical aerosol containing a penetration enhancer padimate O. The volatile compounds in a drug solvent system containing a mixture of volatile and non-volatile solvents as a vehicle can evaporate from the skin creating a supersaturated solution on the skin surface in an attempt to stimulate drug absorption by creating a concentration gradient. One of the major problems of these systems is the difficulty in creating systems that are reproducible since the rate and degree of volatile solvent evaporation will depend upon ambient conditions during application. Variability in absorption kinetics causes fluctuations in drug delivery and unreliable clinical efficacy.

Metered dose devices also require co-ordination and manual dexterity for efficient use. The authors also claim that the dose system provides flexibility and can be moved around to provide a greater surface area of application. Further, it would still be necessary to move the dose around because the enhancer is a hydroxy cinnamate that damages the skin and causes irritation and erythema. Therefore, this aerosol dosing system would offer no more clinical advantage than a patch containing enhancers.

Interest in transdermal drug delivery may be increasing but some fundamental limitations restrict broader application of the technology. The main limitations to use of transdermal deliverys are the rate of transport of the drug through the skin and patient compliance.

Not every drug can be administered transdermally at a rate sufficiently high enough to achieve blood levels that are therapeutically beneficial for systemic medication. Drugs with similar molecular weights and sizes for example may absorb across the skin at different rates. Fentanyl for example permeates the skin at 2 Ig/CM2/hr compared to ephedrine at 200 pg/cm2/hr. The large size of a transdermal delivery system required for fentanyl would therefore be neither practical or economical despite the advantages of the administration route.

Skin enhancers and various formulation techniques have been developed to improve drug absorption through the skin. Skin enhancers can include compounds like capric acid, oleic acid, azone, decylmethyl sulfoxide and hydroxy cinnamates, that typically function to modify structure especially of the stratum corneum by dissolving the lipid matrix to improve permeability of drug compounds.

Dermal absorption of progesterone for example increases by 143% when the stratum corneum is delipidized. The enhancement increases to 843% when the stratum corneum is totally eliminated. With such aggressive modification, commonly reported problems with repeated use of such systems are therefore evident, including contact dermatitis, reddening of the skin, itching and burning that requires movement of the patch, or application of the drug, around the body to prevent local irritation. The reddening is said to disappear within hours of removing the patch. But concern has been raised with respect to long term risk and safety with use of this type of transdermal delivery systems, mainly because increased drug permeability is achieved at the cost of damaging a fundamentally important protective layer of the skin.

Another limitation to transdermal delivery systems is the difficulty in formulating a drug compound that is both sufficiently lipid soluble to absorb through phospholipid membranes yet water soluble to move in the aqueous cytosol and usually hydrophilic suspension media. Drug molecules need to pass both lipophilic and hydrophillic barriers in traversing the skin. This is difficult to achieve and can slow dermal transport.

While improvements in formulation should therefore be considered it is also important to understand that rate of release and bioavailability of drug compounds from transdermal delivery systems can also be dependent upon the device geometry and the nature, thickness and area of the membrane used. Duration of release is believed to be governed by size of the drug reservoir and bioavailability of the drug compound. Mathematical models have been proposed to estimate the kinetics of drug release. Like all physicochemical mathematical approaches, biological factors are not always well described.

Subsequent strategies arising from such models aim to increase solubility and dissolution rate of drugs from dosage forms. Theoretically, these strategies make the drug more available for absorption through membranes and involve techniques such as co-solvent addition, complexation, solid state manipulation of the membrane by skin enhancers and pro-drug modification. However, these mathematical approaches have been criticized for not being able to effectively generate descriptors for three dimensional features, such as hydrophobicity and some electronic effects of drug interaction including hydrogen bonding.

Mathematical descriptors are also inadequate in describing various biological processes including dermal absorption, gastrointestinal absorption, distribution, metabolism and excretion. Current solutions arising from the mathematical model are best described as two dimensional approaches to a three dimensional puzzle.

On this basis, design of current transdermal delivery systems is flawed and cannot account for all important dimensions of passive and active biological transport processes. Current strategies to improve transdermal therapy have not been universally successful and there is scope for further improvement. In particular, there is a need for use of transdermal delivery systems capable of delivering a wider range of drugs. WO 96/21440 A discusses covalent modification of hydroxyl groups of therapeutic agents, to enhance bioavailability.

### Summary of the Invention

It has been found that phosphate derivatives of pharmaceutical with hydroxyl groups surprisingly show faster transport through the skin when compared with the transport of the parent pharmaceutical compound. This leads to useful topical formulations of pharmaceutical such as creams, patches and spray-on forms, that are therapeutically effective and do not risk the integrity of, or damage to, the skin.

According to the first aspect of the invention, there is provided a topical formulation comprising an effective skin-penetrating amount of one or more phosphate derivatives of one or more pharmaceutical hydroxy compounds and acarrier comprising a complex of tocopheryl phosphate and a complexing agent selected from arginine, lysine, histidine and tertiary substituted amines, such as.those according to the following formula:

NR¹R²R³

wherein R¹ is chosen from the group comprising straight or branched chain mixed alkyl radicals from C6 to C22 and carbonyl derivatives thereof; R² and R³ are chosen independently from the group comprising H, CH₂COOX, CH₂CHOHCH₂SO₃X, CH₂CHOHCH₂OPO₃X, CH₂CH₂COOX, CH₂CH₂CHOHCH₂SO₃X or CH₂CH₂CHOHCH₂OPO₃X and X is H, Na, K or alkanolamine, provided R² and R³ are not both H; and wherein when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

In this aspect of the invention the topical formulation is a transdermal delivery system comprising one or more sustained release systems designed to alter absorption kinetics in favor of zero order release.

The term "pharmaceutical hydroxy compounds" is used herein to refer to pharmaceutical active compounds selected from the group consisting of hydroxy compounds where the hydroxy group is not likely to be sterically hindered.

Examples of pharmaceutical hydroxy compounds include but are not limited to narcotic analgesics such as morphine and levorphanol, non narcotic analgesics such as codeine and acetaminophen, corticosteroids such as cortisone, anaesthetics such as propofol, antiemetics such scopolamine, sympathomimetic drugs such as adrenaline and dopamine, antiepileptic drugs such as fosphenytoin, anti-inflammatory drugs such as ibuprofen, thyroid hormones and antithyroid drugs including thyroxine, phytochemicals including a-bisabolol, eugenol, silybin, soy isoflavones, iridoid gylcosides including aucubin and catalpol, sesquiterpene lactones including pseudoguaianolide from Arnica chamissonis, terpenes including rosmarinic acid and rosmanol, phenolic glycosides including the salicylates salicin, saligenin and salicyclic acid, triterpenes taraxasterol or a-lactucerol, and isolactucerol, p-hydroxyphenylacetic acid derivative taraxacoside, hydroquinone derivatives including arbutin, phenylalkanones including gingerols and shogaols, hypercin, and acylphloroglucides including xanthohumol, lupulone, humulone and 2-methylbut-3-en-2-ol.

Specific examples of pharmaceutical hydroxy compounds are as follows:

| | |
|---|---|
| Warfarin and coumarin derivatives | Dienestrol |
| | Epanolol |
| Acetophenazine | Esmolol Estradiol |
| Acyclovir | Estrone |
| Ganciclovir | Ethynylestradiol |
| Indinovir | Ethisterone |
| Lamivudine | Hexestrol |
| Penciclovir | Hydrocortisone |
| Ritinavir | Methylprednisolone saquinavir |
| | methyltestosterone zalsitabine |
| zidovudine | fluconazole |
| Isosorbide mononitrate | dronabinol |
| tacrolimus | Carbidopa |
| Adrenaline | Morphine |
| Chlorprenaline | Apomorphine and other narcotics |
| Atropine | Codeine |
| Denopamine | Naltrexone |
| Dobutamine | Pentazocine |
| Dopamine | phenazocine |
| Epinephrine | tramadol |
| Fenoterol | |
| Formoterol | Atenolol |
| scopolamine | Labetolol |
| | sotolol |
| | timolol |
| Doxefazepam | |
| Lorazepam | Dinoprost |
| temazepam | Epoprostenol Latanoprost |
| terfenadine | misoprostol |
| losartan | doxorubicin Daunorubicin |
| Desmopressin | Paclitaxel |
| Dextrothyroxin | Teniposide |
| Levothyroxine | Topotecan |
| | zorubicin |
| troglitazone | venlafaxine |
| Androstanolone | |
| clocortilone | Atorvastatin |
| Betamethasone | Fluvastatin |
| Cortisone | Lovastatin |
| Danazol | Pravastatin |
| simvastatin | Carbazochrome |
| | Carbuterol |
| | Carfenazine |
| Dipyridamole | Chenodeoxycholic acid |
| | Chloral hydrate |
| Dithranol | Chlorobutanol |
| Calipotriol | Chlorphenesin |
| | Cianidanol |
| | Cimetropium bromide |
| Ambroxol | Clidinium |
| Cefadroxil | Clofoctol |
| Cefamandole | Clopenthixol |
| Cefatrizine | Cyclobutyrol |
| Cefoperazone | Cycrimine |
| Cefpiramide | Cynarine |
| Clavulininc acid | Dezocine |
| Doxycycline | Dichlorophen |
| Latamoxef | Diflunisal |
| Meropenem | Dimercaprol |
| Metacycline | Diprophylline |
| Metronidazole | Dropropizine |
| Minocycline | Edrophonium |
| Novoniocin | Elliptinium |
| Rolitetracycline | Eprozinol |
| Spectinomycin | ergometrine |
| tetracycline | etamivan |
| | etamsylate |
| | ethambutol |
| loperamide | etilifrine |
| | etofenamate |
| Azacosterol | exifone |
| Azacyclanol | fendosal |
| Benzarone | fenpentadiol |
| Benzbromarone | Frenticlor |
| Benziodarone | fentonium |
| Benzilonium | fexofenadine |
| Bicalutamide | flopropione |
| Bifluranol | flupentixole |
| Buclosamide | fluphenazine |
| Buphenine | flutazolam |
| Bupheniode | flutropium |
| Butoctamide | glazionine |
| Butropium | halofantone |
| Butylscopolammonium | homofenazine |
| Cadralazine | hydroxychloroquine |
| Cafaminol | idebenone |
| Cafedrine | ifenprodil |
| Calcitriol | isoxicam |
| Calusteron | ketobemidone |
| Pantethonate | lobeline |
| mazaticol | |
| mazindol | nitroxoline |
| mefloquine | omnipressin |
| meloxicam | oxacoprol |
| mephenesin | oxitriptan |
| meptazinol | oxyphenbutazone |
| metaraminol | piperacetazine |
| mifepristone | pyritinol |
| mitopodozide | quinagolide |
| mitoxantrone | quinidine |
| monobenzene | suplatast tosylate |
| nadolol | tioxolone |
| trifluperidol | |
| trihexyphenidyl (benzhexol) | |
| xipamide | |

Preferably, the pharmaceutical hydroxy compound is selected from the group consisting of estrogen, estradiol, testosterone, atropine, morphine and mixtures thereof.

Atropine is an alkaloid derived from solanaceous plants and is used in treatment of poisoning, where exposure to an organophosphate pesticide, insecticide or herbicide prevents nerve transmission resulting in inhibition of secretions, bradycardia, mydriasis, inhibition of gastrointestinal motility, and excitatory effects similar to adrenaline, which at toxic doses can cause death. Atropine is typically used in this situation as an effective antidote as the alkaloid is a competitive antagonist of acetylcholine at muscarinic receptors, reversing muscarinic effects of the organophosphate exposure. Morphine is also an alkaloid and potent analgesic.

The "phosphate derivatives of pharmaceutical hydroxy compounds" are compounds covalently bound by means of an oxygen to the phosphorus atom of a phosphate group thus forming a carbon-oxygen-phosphorous bond. The oxygen atom is derived from a hydroxyl group on the pharmaceutical hydroxy compounds. The phosphate derivative may exist in the form of a free phosphate acid; a salt thereof; a phosphate ester having two molecules of pharmaceutical hydroxy compounds; a phosphatidyl compound wherein the free phosphate oxygen forms a bond with an alkyl or substituted alkyl group; or a complex with one or more complexing agents selected from the group consisting of amphoteric surfactants, cationic surfactants and amino acids having nitrogen functional groups and proteins rich in these amino acids; or mixtures thereof.

Preferred phosphate derivatives of pharmaceutical hydroxy compounds include oestrogen phosphate ester, estradiol phosphate ester, testosterone phosphate ester, atropine phosphate ester, morphine phosphate ester and mixtures thereof.

For example, a typical mixture of phosphate derivatives of estradiol would include mono-estradiol phosphate derivative and di-estradiol phosphate derivative wherein the amount of mono-estradiol phosphate is no less than equimolar to the amount of di-estradiol phosphate derivative. This mixture of estradiol phosphates may then be complexed with one or more complexing agents selected from the group consisting of amphoteric surfactants, cationic surfactants and amino acids having nitrogen functional groups and proteins rich in these amino acids as disclosed in international patent application no WO 02/40034 A.

The complexing agents are selected from arginine, lysine, histidine and tertiary substituted amines, such as those according to the following formula:

NR¹R²R³

wherein R¹ is chosen from the group comprising straight or branched chain mixed alkyl radicals from C6 to C22 and carbonyl derivatives thereof; R² and R³ are chosen independently from the group comprising H, CH₂COOX, CH₂CHOHCH₂SO₃X, CH₂CHOHCH₂OPO₃X, CH₂CH₂COOX, CH₂CH₂CHOHCH₂SO₃X or CH₂CH₂CHOHCH₂OPO₃X and X is H, Na, K or alkanolamine provided R² and R³ are not both H; and wherein when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

The formation of the complex of the phosphate derivatives of pharmaceutical hydroxy compounds may occur as a separate step in the manufacture process or it may occur in situ in the final topical formulation. For example, the complex of morphine phosphate and lysine may be formed and then added to the carrier to form the topical formulation. Alternatively, morphine phosphate ester may be added to a carrier containing a complexed phosphate entity such as lauryliminodipropionic acid tocopheryl phosphate and there will be some in situ formation of the complex of morphine phosphate and lauryliminodipropionic acid.

Phosphorylation may be accomplished by any suitable method. Preferably, the hydroxyl group-containing compound is phosphorylated using P₄O₁₀ according to the method in international patent application no WO 00/69865 A. Excess diphosphate derivatives may be hydrolyzed using methods known to those skilled in the art.

The term "effective skin-penetrating amount" is used herein to refer to an amount that penetrates the stratum corneum to reach the epidermal and dermal layers of the skin in an amount that is measurably effective in the reduction of one or more symptoms presented by a patient suffering from a skin condition. A person skilled in the art will understand that the actual amount will vary from drug to drug. The effective skin penetrating amount will be sufficient to provide an amount within the therapeutic range of a drug. In some instances, the skin effective penetrating amount will be 1 to 10 times the current therapeutic I.V. dose for a therapeutic drug. For example, an effective skin penetrating amount of estradiol phosphate in a topical formulation according to the invention may be 1.2 times the current I.V. dose of estradiol. For atropine phosphate, the effective skin penetrating amount may be 3 times the current I.V. dose of atropine. If the drug is currently provided orally, then the effective skin penetrating amount may be a fraction of the current therapeutic ingestible dose because ingestible doses have to allow for immediate elimination by the liver.

The topical formulation of the invention may be any suitable topically applied delivery systems considered by those skilled in the pharmaceutical art as capable of delivering drugs topically on human or other animal skin to achieve a systemic or dermal effect. It includes but is not limited to creams, lotions, gels, emulsions, liposomes, aerosols, patches, poultices, subcutaneous depots, plasters and sustained release systems designed to alter absorption kinetics in favor of zero-order release.

The term "transdermal delivery system" is used herein to refer to sustained release systems designed to alter absorption kinetics in favor of zero-order release.

The term "acceptable carrier" is used herein to refer to a carrier considered by those skilled in the pharmaceutical art to be useful in topical formulations and transdermal delivery systems capable of being used topically on human or other animal skin.

Thecarrier for a topical formulation according to the invention comprises a complex of tocopheryl phosphates and acomplexing agents selected from arginine, lysine, histadine and tertiary substituted amines, such as those according to the following formula:

NR¹R²R³

wherein R¹ is chosen from the group comprising straight or branched chain mixed alkyl radicals from C6 to C22 and carbonyl derivatives thereof;

R² and R³ are chosen independently from the group comprising H, CH₂COOX, CH₂CHOHCH₂SO₃X, CH2CHOHCH₂OPO₃X, CH₂CH₂COOX, CH₂CH₂CHOHCH₂SO₃X or CH₂CH₂CHOHCH₂OPO₃X and X is H, Na, K or alkanolamine provided R² and R³ are not both H; and and wherein when R¹ is RCO then R² may be CH₃ and R³ may be (CH₂CH₂)N(C₂H₄OH)-H₂CHOPO₃ or R² and R³ together may be N(CH₂)₂N(C₂H₄OH)CH₂COO-.

Preferably, the carrier comprises lauryliminodipropionic acid tocopheryl phosphate.

Other excipients such as skin penetration enhancers, solvents, surfactants emollients, preservatives colorants and fragrances can be additionally used. A person skilled in the art will know what components may be used as the additional carrier for the formulations of the present invention. Conventional pharmaceutical processes may be used in making up these common forms of compositions.

A typical carrier for a topical formulation according to the invention comprises 61.95% deionized water, 5.00% glycerin, 0.05% trisodium EDTA, 0.50% carbomer (Carbopol Ultrez 10), 7.50% lauryliminodipropionic acid tocopheryl phosphate, 2.00% Phoenoxol T (cetearyl alcohol and ceteareth-20), 1.00% glyceryl stearate (Emerest 2400), 5.00% isopropyl myristate (Pelemol IPM), 3.50% cetyl ethylhexanoate (Pelemol 168), 3.50% isocetyl behenate (Pelemol ICB), 3.00% oleyl erucate (Cetiol J-600), 0.50% dimethicone (Dow 200,100 cSt. ), 5.00% deionized water, 0.50% triethanolamine (99%) and 1.00% Germaben II (propylene glycol, diazolidinyl urea, methylparaben and propylparaben).

The topical compositions of the invention can be used for improving skin absorption and/or maintaining adequate blood levels for prolonged periods of thepharmaceutical hydroxy compound.

In one aspect of the invention the pharmaceutical hydroxy compound is estrogen. The corresponding topical formulation can be used for hormone replacement therapy.

Advantages of the present invention relates to the surprising absorption (movement) through the skin of the pharmaceutical hydroxy compound, effectiveness and longer timeframe that a transdermal delivery system can be utilised on a subject.

According to a further aspect of the invention, there is provided a transdermal delivery system comprising:
(a) a device for applying a pharmaceutical composition to the skin of a subject; and
(b)a topical formulation of the invention in the device.

Preferably, the device is a patch, poultice, gel, cream, plaster or other sustained-release system designed to alter absorption kinetics in towards zero order release.

The one or more phosphate derivatives of a pharmaceutical hydroxy compound may be in admixture with one or more suitable diluents or excipients.

The pharmaceutical hydroxy compound can be administered to a subject byapplying the transdermal delivery system of the present invention to a portion of skin of the subject such that the one or more phosphate derivatives of the pharmaceutical hydroxy compound are transported through the skin barrier of the subject and delivered to the blood stream.

The present inventors have found that the phosphate derivatives of pharmaceutical hydroxy compounds effectively permeate the dermal layers significantly more than non-phosphated pharmaceutical hydroxy compounds when used without skin enhancers. As there is no need for skin enhancers, there is no skin irritation or erythema when phosphate derivatives of pharmaceutical hydroxy compounds are used.

In order that the present invention may be more clearly understood, preferred forms will be described with reference to the following drawings and examples.

### Brief Description of the Drawings

Figure 1: Changes in total estrogens (mean ± SD) measured in plasma samples obtained from ovariectomised hairless rats to which formulations containing approximately 0. 17µg of estrogen (E) or estrogen phosphate (EP) were applied.
Figure 2: Percent change in tritiated E vs tritiated EP in ovariectomised hairless rats to which formulations containing tritiated E or EP were applied.
Figure 3: Changes in total testosterone (mean ± SD) measured in plasma samples obtained from ovariectomised hairless rats to which formulations containing approximately 1.00 µg ± 0.02 µg of T or TP were applied.

### Examples

The invention is further explained and illustrated by the following non-limiting examples.

### Example 1 (Reference Example)

Twelve grams (0.03 g/mole) of disodium-N-lauryl beta imino dipropionate were dissolved in 88 grams of distilled water to provide a 12% wt/wt clear solution with pH 12. Morphine-3-phosphoric acid ester (11.43 grams (0.03 g/mole)) was slowly added and mixed until uniform. The resulting product was a complex consisting of N-lauryl beta imino dipropionate-morphine (3) phosphate as a 21.03% wt/wt aqueous dispersion. This complex product was formulated via dilution with water preservative buffers together with gelling agents and applied to the skin to elicit transdermal drug delivery.

The complex product may be modified as needed by increasing or decreasing the molar ratio of the disodium-N-lauryl beta imino dipropionate.

Example 2 The transdermal delivery of estradiol and estradiol phosphate in the hairless rat model was evaluated in this example.

Methods Animals : 11 female albino hairless rats were ovariectomised under isoflurane- induced anaesthesia and allowed to recover for 10 days prior to experimentation.

This allowed clearance of any estrogens from the body.

Blood sampling : Blood samples (500 NI) were obtained from the tail vein of conscious restrained rats at 0,1, 2,4, 8,16 and 24 hours following application of both the estradiol (n=5) and estradiol phosphate (n=6) formulations. Blood was collected into EDTA tubes, then centrifuged at 5000 rpm for 10 minutes. Plasma was removed and stored at -80°C until assayed.

*Transdermal Formulation Preparation and Application:* estradiol and estradiol phosphate were provided by Tocovite Pty Ltd and prepared at concentrations of 20 µg/ml approximately 1 hour before application in a carrier cream prepared as follows:

| **PHASE A** | **W/W** |
|---|---|
| Deionized water | 61.95% |
| Glycerin | 5.00 |
| Trisodium EDTA | 0.05 |
| Carbomer (Carbopol Ultrez 10)² | 0.50 |
| Lauryliminodipropionic Acid Tocopheryl Phosphate' | 7.50 |

| **PHASE B** | |
|---|---|
| Cetearyl Alcohol (and) Ceteareth-20 (Phoenoxol T)³ | 2.00 |
| Glyceryl Stearate (Emerest 2400)⁴ | 1.00 |
| Isopropyl Myristate (Pelemol IPM)³ | 5.00 |
| Cetyl Ethylhexanoate (Pelemol 168)³ | 3.50 |
| Isocetyl Behenate (Pelemol ICB)³ | 3.50 |
| Oleyl Erucate (Cetiol J-600)⁴ | 3.00 |
| Dimethicone (Dow 200,100 cSt.)⁵ | 0.50 |

| **PHASE C** | |
|---|---|
| Deionized Water | 5.00 |
| Triethanolamine (99%) | 0.50 |

| **PHASE D** | |
|---|---|
| Propylene Glycol (and) Diazolidinyl Urea (and) Methylparaben (and) Propylparaben (Germaben II)⁶ | 1.00 |
| | 100.00% |

| | |
|---|---|
| 1. Vital Personal Care, Incorporated 2. B.F. Goodrich, Incorporated 3. Phoenix Chemical, Incorporated 4. Cognis, Incorporated , 5. Dow-Corning, Incorporated 6. ISP Corporation | |

Procedure: Combine Phase A items minus the carbomer and lauryliminodipropionic acid tocopheryl phosphate. When a solution is obtained, disperse carbomer in this solution. Begin heating Phase A to 70-75°C with adequate agitation. Disperse lauryliminodipropionic acid tocopheryl phosphate in carbomer mucilage with sweep agitation. Combine Phase B items and heat to 75-80°C with adequate agitation. With Phase A is uniform and at 70-75°C and Phase B is uniform and at 75-80°C, add Phase B to Phase A with adequate agitation. Allow AB to cool to 50°C and then add Phase C solution to AB. Continue adequate agitation of ABC until 45°C is reached. Add Phase D to ABC. Continue adequate agitation until 35°C is reached.

Estradiol Phosphate (***EP***): 4.3 mg of EP was dissolved in 17.3 ml of acetone (0.25 mg/ml). Twenty µl was transferred to an Eppendorf tube and the solvent was evaporated in a nitrogen stream. Then 0.999 g of the carrier cream was added, and mixed with a glass rod and centrifuged. This was repeated 5 times. Final concentration = 4.90 µg/ml. (composition according to the invention)

Estradiol (***E***): 6.7 mg was dissolved in 26.8 ml of absolute ethanol (0.25 mg/ml). Twenty µL was transferred to an Eppendorf tube and the solvent was evaporated in a nitrogen stream. Then 1.003 g of the carrier cream was added, and mixed with a glass rod and centrifuged. This was repeated 5 times. Final concentration = 4.89 pg/ml. (composition not according to the invention)

Each formulation was applied to the dorsal skin of an anaesthetised rat in an area of approximately 4 cm² marked with an indelible felt tip marker. Application of approximately 30 mg of formulation (containing 0. 17 µg of E or EP) was applied to the site with a curved glass rod applicator. The formulation was 'rubbed' in until it appeared to have been absorbed into the skin, which took between 5-10 min. Any changes in the consistency of the formulation during this procedure were noted.

The amount of formulation applied and the area of the application site were weighed for each animal.

*Organ Collection:* After 24 hr monitoring animals were killed with an overdose of anaesthetic. All organs were removed, weighed and stored at -80°C until assay.

*Total Estrogens RIA:* The RIAs were performed using a commercially available total estrogens kit (ICN Pharmaceuticals, catalogue # 07-140205) with 100% cross-reactivities for 17β-estradiol and estrone. The standard curve range for this assay is 2.5-100 pg/ml (r²= -0. 943). Extraction efficiency was determined through a series of spiking assays and was between 90 to 98% using diethyl ether as the extraction solvent for rat plasma and organs. This solvent did not interfere with the assay. Plasma volumes of 100 µl were used for assay.

### Results

*Formulation Application:* The average areas (± SE) to which formulations were applied on the dorsum of the animals were 3.88 ± 0.03 cm² and 3.88 ± 0.07 cm² for the E and EP groups respectively. The average amounts of these formulations applied in the E and EP groups were 0.17 µg.

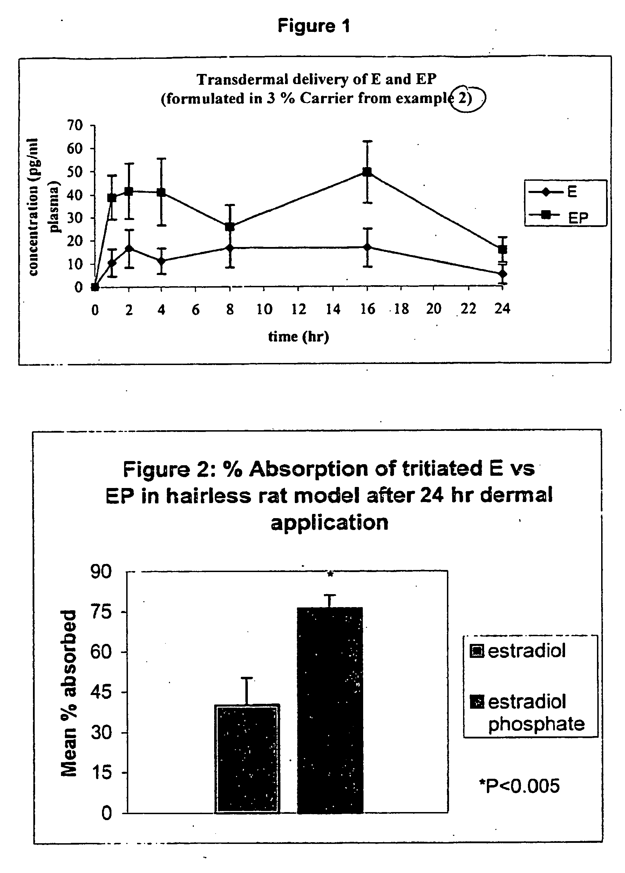

*Total Estrogens in Plasma:* Measurable levels of estrogens (between the standard curve range of 2.5 - 100 pg/ml) were present in both groups of animals with maximum concentrations of 16.63 ± 8.18 (mean ± SE) pg/ml plasma measured in the E group at 2, 8 and 16 hr post-application and a maximum concentration of 49.16 ± 13.21 pg/ml plasma measured at 16 hr post-application in the EP group (Figure 1). Baseline measurements taken at t=0 were subtracted from all values to correct for background levels present in the plasma.

### Discussion

This study evaluated the transdermal delivery of EP and E in female hairless rats. The concentration of estradiol in blood was consistently higher when estradiol phosphate was applied over a 24-hour period (statistically significant, *P<0.01* at 2, 4 and 16 hours). At the equivalent doses that were applied the EP resulted in at least twice the plasma concentration of the hormone compared to the E treatment. This suggests that EP formulated may provide a more effective formulation for delivering E.

### Conclusion

The trial demonstrated that useful doses of estradiol may be delivered based on the hairless rat model and it may be inferred from the similarity of the properties of the hairless rat to human skin. Even more surprising, however, was the finding that EP delivered free estradiol in circulating blood and thus estradiol phosphate may prove to be efficacious for hormone replacement therapy.

### Example 3

The acute transdermal penetration of ³H-Estradiol (³H-E) and ³H-Estradiol Phosphate (³H-EP) in the hairless rat model was evaluated in this model.

### Methods

*Animals*: 6 female albino hairless rats were used in this study (n=3 per treatment group).

*Transdermal Formulation Preparation and Application:* ³H-E and ³H-EP were provided by Tocovite Pty Ltd and prepared in formula approximately 1 hr before application in the carrier cream used in Example 2.

Twenty µl of ³H-E and ³H-EP were aliquoted into 1 ml Eppendorf tubes. The solvents from both ³H-E and ³H-EP were evaporated under a stream of nitrogen. Once completely dry 0.498 g of the carrier cream was added to ³H-E and 0.502 g to ³H-EP and mixed with a glass rod and centrifuged for 1 minute. This was repeated 5 times.

Each formulation was applied to the dorsal skin of an anaesthetised rat in an area of approximately 4 cm² marked with an indelible felt tip marker. Application of approximately 30 mg of formulation (containing 5 pg of ³H-E (not according to the invention) and ³H-EP (according to the invention)) was applied to the site with a curved glass rod applicator. The formulation was 'rubbed' in until it appeared to have been absorbed into the skin, which took between 5 to 10 minutes. A tegaderm (3M) patch was applied to the area to prevent animals from removing the formulation.

### Results and Discussion

This study clearly demonstrated that EP was more readily absorbed in comparison to E when transdermally applied (Figure 2). Analysis of individual skin layers was also undertaken in this study and revealed that minimal E or EP remained in the skin 24 hours after application. Higher levels of EP were found in the epidermis and dermis due to higher volumes of the EP moving through the skin during the 24-hour period. This suggests that more of the EP was transported through the skin during the treatment period.

### Example 4

The transdermal delivery of testosterone and testosterone phosphate in the hairless rat model was investigated in this example.

### Methods

*Animals:* 12 Female albino hairless rats were ovariectomised under isofurane- induced anaesthesia and allowed to recover for 15 days prior to experimentation.

*Blood Sampling:* Blood samples (500 µl) were obtained from the tail vein of conscious restrained rats at 0, 1, 2, 4, 8, 16 and 24 hr following application of both testosterone (n=6) and testosterone phosphate (n=6) formulations. Blood was collected into EDTA tubes, then centrifuged at 5000 rpm for 10 min. Plasma was removed and stored at-80°C until assay.

*Transdermal Formulation Preparation and Application:* Testosterone and testosterone phosphate were provided by Tocovite Pty Ltd and prepared in the carrier cream used in Example 2 approximately 1 hr before application.

Testosterone Phosphate (TP): 4.41 mg of TP was dissolved in 15 ml of water and then made up to 100 ml with ethanol. 1 ml was transferred to an Eppendorf tube and the solvent was.evaporated under a nitrogen stream. 1.00 g of the carrier cream from Example 2 was added and mixed with a glass rod and centrifuged. This was repeated 5 times. (composition according to the invention)

Testosterone (***T***): 3.94 mg of T was dissolved in 15 ml of water and then made up to 100 ml with ethanol. 1 ml was transferred to an Eppendorf tube and the solvent was evaporated under a nitrogen stream. 1.00 g of the carrier cream from Example 2 was added and mixed with a glass rod and centrifuged. This was repeated 5 times.

### (composition not according to the invention).

Each formulation was applied to the dorsal skin of an anaesthetized rat in an area of approximately 4 cm² marked with an indelible felt tip marker. Application of approximately 30 mg of formulation (containing 1 µg of T or TP) was applied to the site with a curved glass rod applicator. The formulation was 'rubbed' in until it appeared to have been absorbed into the skin, which took between 5 to 10 min. Any changes in the consistency of the formulation during this procedure were noted.

*Results Formulation Application:* The average amounts of these formulations applied in the T and TP groups were 1 µg ± 0.02 µg.

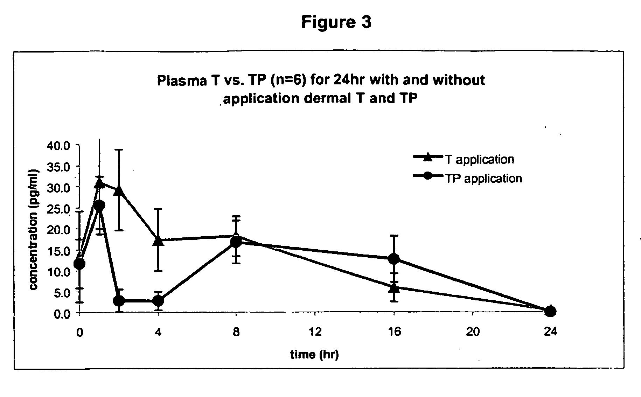

*Total Testosterone in Plasma:* Measurable levels of testosterone (between standard curve range 2.5-100 pg/ml) were present in both groups of animals with maximum concentrations of 30.90 ± 11. 00 (mean ± SD) pg/ml plasma measured in the T groups at 1,8 and 16 hr post-application. Baseline measurements were taken at t=0 and these values were subtracted from all values to correct for background levels in the plasma (Figure 3).

### Discussion

The concentration of testosterone in blood increased when both testosterone and testosterone phosphate was applied.

### Example 5

The efficacy of atropine phosphate when compared to atropine sulfate was investigated in this example.

Atropine sulfate (not according to the invention) and atropine phosphate (according to the invention) formulated in phosphate buffer and given intravenously (I.V.) each to two rats at 2mg/kg resulted similar increases in heart rates.

For example, the baseline heart rate of a rat was approx. 350 bpm and increased to 450 bpm within 15 minutes after administration of atropine (either atropine phosphate or atropine sulfate). Approximately two hours later, normal heart rates were restored. This is regarded as a significant change in pulse rate.

These results show that atropine phosphate is effective to increase pulse rate. It is expected that when provided transdermally similar results will be achieved.

## Claims

1. A topical formulation comprising an effective skin-penetrating amount of one or more phosphate derivatives of one or more pharmaceutical hydroxy compounds, wherein the pharmaceutical hydroxy compound is covalently bound by means of an oxygen atom of the hydroxy group to a phosphorous atom of a phosphate group to form a carbon-oxygen-phosphorous bond, and a carrier comprising a complex of tocopheryl phosphate and a complexing agent selected from arginine, lysine, histidine and tertiary substituted amines, such as those according to the following formula:
NR¹R²R³
wherein R¹ is chosen from the group comprising straight or branched chain mixed alkyl radicals from C6 to C22 and carbonyl derivatives thereof;
R² and R³ are chosen independently from the group comprising H, CH₂COOX, CH₂CHOHCH₂SO₃X, CH₂CHOHCH₂OPO₃X, CH₂CH₂COOX, CH₂CH₂CHOHCH₂SO₃X or CH₂CH₂CHOHCH₂OPO₃X and X is H, Na, K or alkanolamine, provided R² and R³ are not both H; and
wherein when R¹ is RCO then R² may be CH₃ and R³ may be (CH₂CH₂)N(C₂H₄OH)-H₂CHOPO₃ or R₂ and R₃ together may be N(CH₂)₂N(C₂H₄OH)CH₂COO-.

2. A topical formulation according to claim 1 wherein the topical formulation is a transdermal delivery system comprising one or more sustained release systems designed to alter absorption kinetics in favor of zero-order release.

3. A topical formulation according to either claim 1 or 2 wherein the pharmaceutical hydroxy compound is selected from the group consisting of estrogen, estradiol, testosterone, atropine, morphine and mixtures thereof.

4. A topical formulation according to claim 1 wherein the phosphate derivatives of pharmaceutical hydroxy compounds are selected from estrogen phosphate ester, estradiol phosphate ester, testosterone phosphate ester, atropine phosphate ester, morphine phosphate ester and mixtures thereof.

5. A topical formulation according to claim 1 wherein the carrier comprises lauryliminodipropionic acid tocopheryl phosphate.

6. A topical formulation according to claim 5 wherein the carrier comprises 61.95% deionized water, 5.00% glycerin, 0.05% trisodium EDTA, 0.50% carbomer, 7.50% lauryliminodipropionic acid tocopheryl phosphate, 2.00% cetearyl alcohol and ceteareth-20, 1.00% glyceryl stearate, 5.00% isopropyl myristate, 3.50% cetyl ethylhexanoate, 3.50% isocetyl behenate, 3.00% oleyl erucate, 0.50% dimethicone, 5.00% deionized water, 0.50% triethanolamine (99%) and 1.00% propylene glycol, diazolidinyl urea, methylparaben and propylparaben.

7. A transdermal delivery system comprising:
(a) a device for applying a pharmaceutical composition to the skin of a subject; and
(b) a topical composition as defined in any of claims 1 to 6 in the device.

8. A transdermal delivery system according to claim 7 wherein the device is a patch, poultice, gel, cream, plaster or other sustained-release system designed to alter absorption kinetics in towards zero-order release.

9. A topical formulation as defined in any of claims 1 to 6 for use in improving the skin absorption and/or maintaining adequate blood levels for prolonged periods of the pharmaceutical hydroxy compound.

10. A topical formulation as defined in any of claims 1 to 6 wherein the pharmaceutical hydroxy compound is estrogen and wherein the topical formulation is for use in hormone replacement therapy.

11. Use of a carrier for the manufacture of a topical formulation wherein the topical formulation is as defined in any of claims 1 to 6 and wherein the topical formulation is for use in improving the skin absorption and/or maintaining adequate blood levels for prolonged periods of the pharmaceutical hydroxy compound.

12. Use of a carrier for the manufacture of a topical formulation wherein the topical formulation is as defined in any of claims 1 to 6, wherein the pharmaceutical hydroxy compound is estrogen and wherein the topical formulation is for use in hormone replacement therapy.

## Patentansprüche

1. Eine topische Formulierung umfassend eine wirksame Haut penetrierende Menge von einem oder mehreren Phosphatderivaten von einer oder mehreren pharmazeutischen Hydroxyverbindungen, wobei die pharmazeutische Hydroxyverbindung kovalent, mittels eines Sauerstoffatoms der Hydroxygruppe an ein Phosphoratom einer Phosphatgruppe gebunden ist, um eine Kohlenstoff-Sauerstoff-Phosphor Bindung auszubilden, und einen Träger der einen Komplex von Thocopheryl-Phosphat und einem Komplexbildner ausgewählt aus Arginin, Lysin, Histidin und tertiären substituierten Aminen umfasst, wie die der nachfolgenden Formel:
NR¹R²R³
wobei R¹ ausgewählt ist aus der Gruppe, welche geradkettige oder verzweigte gemischte Alkylreste von C6 bis C22 und Carbonylderivate davon umfasst; R² und R³ unabhängig voneinander ausgewählt sind aus der Gruppe, welche H, CH₂COOX, CH₂CHOHCH₂SO₃X, CH₂CHOHCH₂OPO₃X, CH₂CH₂COOX, CH₂CH₂CHOHCH₂SO₃X oder CH₂CH₂CHOHCH₂OPO₃X umfasst und X = H, Na, K oder Alkanolamin ist, mit der Maßgabe, dass R² und R³ nicht gleichzeitig H sind; und wobei, wenn R¹ RCO ist, dann R² CH₃ sein kann und R³ (CH₂CH₂)N(C₂H₄OH)-H₂CHOPO₃ sein kann oder R² und R³ zusammen N(CH₂)₂N(C₂H₄OH)CH₂COO- sein können.

2. Eine topische Formulierung gemäß Anspruch 1, wobei die topische Formulierung ein System zur Verabreichung durch die Haut ist, welches ein oder mehr Systeme mit verzögerter Freisetzung umfasst, die vorgesehen sind die Absorptions Kinetik zu Gunsten einer Freisetzung nullter Ordnung zu verändern.

3. Eine topische Formulierung nach einem der Ansprüche 1 oder 2, wobei die pharmazeutische Hydroxyverbindung ausgewählt ist aus der Gruppe, bestehend aus Östrogen, Estradiol, Testosteron, Atropin, Morphin und Gemischen davon.

4. Eine topische Formulierung gemäß Anspruch 1, wobei die Phosphatderivate von pharmazeutischen Hydroxyverbindungen ausgewählt sind aus Östrogenphosphatester, Estradiolphosphatester, Testosteronphosphatester, Atropinphosphatester, Morphinphosphatester und Gemischen davon.

5. Eine topische Formulierung gemäß Anspruch 1 wobei der Träger Lauryliminodipropionsäuretocopherylphosphat umfasst.

6. Eine topische Formulierung gemäß Anspruch 5, wobei der Träger 61,95% entionisiertes Wasser, 5,00% Glycerin, 0,05% Trinatrium EDTA, 0,50% Carbomer, 7,50% Lauryliminodipropionsäuretocopherylphosphat, 2,00% Cetearylalkohol und Ceteareth-20, 1,00% Glycerylstearat, 5,00% Isopropylmyristat, 3,50% Cetylethylhexanoat, 3,50% Isocetylbehenat, 3,00% Oleylerucat, 0,50% Dimethicon, 5,00% entionisiertes Wasser, 0,50% Triethanolamin (99%) und 1,00% Propylenglykol, Diazolidinylharnstoff, Methylparaben und Propylparaben umfasst.

7. Ein System zur Verabreichung durch die Haut umfassend:
(a) eine Vorrichtung zum Auftragen eines Arzneimittels auf die Haut eines Individuums; und
(b) eine topische Zusammensetzung gemäß einem der Ansprüche 1 bis 6 in der Vorrichtung.

8. Ein System zur Verabreichung durch die Haut gemäß Anspruch 7 wobei die Vorrichtung ein Pflaster, Maske, Gel, Creme, Heftpflaster oder ein anderes System mit verzögerter Freisetzung, das vorgesehen ist die Absorptions Kinetik in Richtung einer Freisetzung nullter Ordnung zu verändern, ist.

9. Eine topische Formulierung wie definiert in einem der Ansprüche 1 bis 6, zur Verwendung bei der Verbesserung der Hautabsorption und/oder Aufrechterhaltung adäquater Blutspiegel der pharmazeutischen Hydroxyverbindung über längere Zeit.

10. Eine topische Formulierung wie definiert in einem der Ansprüche 1 bis 6, wobei die pharmazeutische Hydroxyverbindung Östrogen ist und wobei die topische Formulierung zur Verwendung in der Hormonersatztherapie ist.

11. Verwendung eines Trägers zum Herstellen einer topischen Formulierung, wobei die topische Formulierung definiert ist wie in einem der Ansprüche 1 bis 6 und wobei die topische Formulierung zur Verwendung bei der Verbesserung der Hautabsorption und/oder Aufrechterhaltung adäquater Blutspiegel der pharmazeutischen Hydroxyverbindung über längere Zeit ist.

12. Verwendung eines Trägers zum Herstellen einer topischen Formulierung, wobei die topische Formulierung definiert ist wie in einem der Ansprüche 1 bis 6, wobei die pharmazeutische Hydroxyverbindung Östrogen ist und wobei die topische Formulierung zur Verwendung in der Hormonersatztherapie ist.

## Revendications

1. Formulation topique comprenant une quantité efficace pénétrant la peau d'un ou plusieurs dérivés phosphates d'un ou plusieurs composés pharmaceutiques hydroxy, dans laquelle le composé pharmaceutique hydroxy est lié de manière covalente par l'intermédiaire d'un atome d'oxygène du groupe hydroxy à un atome de phosphore d'un groupe phosphate pour former une liaison carbone-oxygène-phosphore, et un support comprenant un complexe de phosphate de tocophéryle et un agent complexant choisi parmi l'arginine, la lysine, l'histidine et des amines tertiaires substituées, comme celles selon la formule suivante :
NR¹R²R³
où R¹ est choisi dans le groupe comprenant les radicaux alkyles en C6-C22 mélangés à chaîne linéaire ou ramifiée et des dérivés carbonyles de ceux-ci ;
R² et R³ sont choisis indépendamment dans le groupe comprenant H, CH₂COOX, CH₂CHOHCH₂SO₃X, CH₂CHOHCH₂OPO₃X, CH₂CH₂COOX, CH₂CH₂CHOHCH₂SO₃X ou CH₂CH₂CHOHCH₂OPO₃X et X est H, Na, K ou alcanolamine, lesdits R² et R³ n'étant pas tous les deux H ; et
où quand R¹ est RCO alors R² peut être CH₃ et R³ peut être (CH₂CH₂)N (C₂H₄OH)-H₂CHOPO₃ ou R² et R³ peuvent être conjointement N(CH₂)₂N (C₂H₄OH)CH₂COO-.

2. Formulation topique selon la revendication 1, ladite formulation topique étant un système d'administration transdermique comprenant un ou plusieurs systèmes à libération prolongée conçus pour modifier la cinétique d'absorption en faveur de la libération d'ordre zéro.

3. Formulation topique selon la revendication 1 ou 2, dans laquelle le composé pharmaceutique hydroxy est choisi dans le groupe constitué de l'oestrogène, de l'oestradiol, de la testostérone, de l'atropine, de la morphine et des mélanges de celles-ci.

4. Formulation topique selon la revendication 1, dans laquelle les dérivés phosphates des composés pharmaceutiques hydroxy sont choisis parmi l'ester de phosphate de l'oestrogène, l'ester de phosphate de l'oestradiol, l'ester de phosphate de la testostérone, l'ester de phosphate de l'atropine, l'ester de phosphate de la morphine et des mélanges de celles-ci.

5. Formulation topique selon la revendication 1, dans laquelle le support comprend le phosphate de tocophéryle de l'acide lauryliminodipropionique.

6. Formulation topique selon la revendication 5, dans laquelle le support comprend 61,95% d'eau désionisée, 5,00% de glycérine, 0,05% d'EDTA trisodique, 0,50% de carbomère, 7,50% de phosphate de tocophéryle de l'acide lauryliminodipropionique, 2,00% d'alcool cétéarylique et de ceteareth-20, 1,00% de stéarate de glycéryle, 5,00% de myristate d'isopropyle, 3.50% d'éthylhexanoate de cétyle, 3,50% de béhénate d'isocétyle, 3,00% d'érucate d'oléyle, 0,50% de dimethicone, 5,00% d'eau désionisée, 0,50% de triéthanolamine (99%) et 1,00% de propylène glycol, d'urée de diazolidinyl, de méthylparabène et de propylparabène.

7. Système d'administration transdermique comprenant:
(a) un dispositif pour appliquer une composition pharmaceutique à la peau d'un sujet ; et
(b) une composition topique comme définie dans l'une quelconque des revendications 1 à 6 dans le dispositif.

8. Système d'administration transdermique selon la revendication 7, dans lequel le dispositif est un patch, un cataplasme, un gel, une crème, un pansement ou autre système à libération prolongée conçu pour modifier la cinétique d'absorption vers la libération d'ordre zéro.

9. Formulation topique selon l'une quelconque des revendications 1 à 6 pour utilisation dans l'amélioration de l'absorption par la peau et/ou maintien des niveaux adéquats dans le sang du composé pharmaceutique hydroxy pendant des périodes prolongées.

10. Formulation topique selon l'une quelconque des revendications 1 à 6, dans laquelle le composé pharmaceutique hydroxy est l'oestrogène et dans laquelle la formulation topique est destinée à une utilisation dans la thérapie de remplacement d'hormone.

11. Utilisation d'un support pour la fabrication d'une formulation topique, dans laquelle ladite formulation topique est comme définie dans l'une quelconque des revendications 1 à 6 et dans laquelle la formulation topique est destinée à une utilisation dans l'amélioration de l'absorption par la peau et/ou maintien des niveaux adéquats dans le sang du composé pharmaceutique hydroxy pendant des périodes prolongées.

12. Utilisation d'un support pour la fabrication d'une formulation topique, dans laquelle la formulation topique est comme définie dans l'une quelconque des revendications 1 à 6, dans laquelle le composé pharmaceutique hydroxy est l'oestrogène et dans laquelle la formulation topique est destinée à une utilisation dans la thérapie de remplacement d'hormone.
